Europäisches Patentamt

European Patent Office

Office européen des brevets

(19)

(11)  EP 0 067 459 B2

(12)  NEW EUROPEAN PATENT SPECIFICATION

(45) Date of publication and mention
of the opposition decision:
**07.10.1998  Bulletin 1998/41**

(45) Mention of the grant of the patent:
**09.03.1988  Bulletin 1988/10**

(21) Application number: **82200382.8**

(22) Date of filing: **29.03.1982**

(51) Int Cl.[6]: **C01B 33/16, B01J 21/08**

(54) **Silica particles and method for their preparation**

Siliciumdioxidteilchen und Verfahren zu ihrer Herstellung

Particules en silice et procédé de production

(84) Designated Contracting States:
**BE DE FR GB IT NL SE**

(30) Priority: **13.04.1981  GB 8111587**

(43) Date of publication of application:
**22.12.1982  Bulletin 1982/51**

(73) Proprietor: **SHELL INTERNATIONALE
RESEARCH MAATSCHAPPIJ B.V.
2596 HR Den Haag (NL)**

(72) Inventors:
• **Spek, Theo Gerard
NL-1031 CM Amsterdam (NL)**
• **Van Beem, Martinus Jan Lodewijk
NL-1031 CM Amsterdam (NL)**

(74) Representative: **Aalbers, Onno et al
P.O. Box 162
2501 AN Den Haag (NL)**

(56) References cited:
DE-A- 1 792 601          DE-A- 2 549 411
DE-A- 2 633 198          DE-A- 2 719 055
DE-A- 2 753 392          DE-A- 2 753 393
DE-B- 2 127 649          DE-C- 1 266 741
FR-A- 1 473 239          FR-A- 1 473 240
FR-A- 1 482 867          US-A- 3 652 215

• **IND. ENG. CHEM., PROD. RES. DEV., vol. 14, no.
3, 1975, T.P. KOBYLINSKI et al. "Porous silica
beads- A catalyst support for removal of exhaust
gas pollutants", pp. 147-150**
• **Journal of Chromatography, 83 (1973) p. 5-9**
• **Unger "Porous Silica" p. 169-172**
• **ASTM D 4058 - 92**
• **ILER, "The Chemistry of Silica", p. 40-43**

Remarks:
The file contains technical information submitted
after the application was filed and not included in this
specification

Printed by Jouve, 75001 PARIS (FR)

## Description

The present invention relates to a process for the preparation of silica particles especially silica spheres having improved performance as catalysts. The present invention also relates to silica particles with a narrow pore diameter distribution, especially silica spheres and to catalysts made therefrom.

Silica particles are applied on a large scale, for example, as catalysts, catalyst carriers, absorbents, drying agents and ion exchangers. For most of these applications globular particles of uniform shape with a high crushing strength and preferably also with a high water resistance are desired. A well-known technique for preparing such particles comprises the so-called sol-gel method, as described in Dutch laid-open patent specification 6,914,492 and German patent specification 1,266,741. This method comprises the initial preparation of a silica hydrogel by mixing an aqueous solution of an alkalimetal silicate with an aqueous solution of an acid, converting the hydrosol obtained into droplet form and gelling the droplets in a liquid which is not miscible with water. The hydrogel obtained is then converted into a xerogel by reducing the alkalimetal content of the globular silica hydrogel particles to less than 1%w, calculated on dry material, followed by drying and calcining.

Although many processes have been described in the art related to various aspects of the successive steps n the conversion of the initial hydrogel into the final xerogel, a problem remaining over the years comprises the inflexibility of the process with respect to size and uniformity of the silica particles concerned. It would be of great value when a process for the manufacture of silica particles could be developed which is not only flexible in that silica particles, especially spherical particles of various sizes could be produced in different batches but also flexible in that the structure parameters pore volume and pore diameter can be set in a wide range rather than producing a product having volume/diameter ratios which cannot be varied independently.

It is known from German Patent Application 2633198 to prepare a silica, suitable for use in high pressure-chromatography, especially gel-chromatography. Before being subjected to a hydrothermal treatment, the silica hydrogel is subjected to a pre-drying step whereby the removal of water is complete, since very stringent drying conditions are applied. When the obtained silica gel is again brought into contact with water the silica gel will deteriorate and have sizes below 100 μm, e.g. from 30 to 70 μm. For the objects of our invention the above-described method in German Patent Application 2633198 is inadequate, since in the preparation of silica according to the inventive method the amount of fines must be very low and secondly it must be possible to define the pore volume and the pore diameter independently of each other.

Furthermore FR-A-14 75 929 discloses silica particles having a porosity similar to the silica particles of the invention.

It has now been found that by carefully choosing the conditions during the conversion of the hydrogel into the final xerogel, silica particles of different sizes can be produced at different times which moreover exhibit an extremely well-defined and settable pore volume and pore diameter. The properties of the silica particles thus prepared render them very suitable for use as catalyst carriers in many chemical and physical processes such as hydrodemetallization, ethanol synthesis and the co-production of styrene and propylene oxide. It is the surprising feature of the present invention that carriers specific for a wide range of applications can be prepared using the same process by carefully adjusting the process conditions.

The present invention therefore relates to a process for the preparation of silica particles by:

a) preparing a silica hydrosol by mixing an aqueous solution of an alkalimetal silicate with an aqueous solution of an acid,
b) converting the hydrosol into droplet form,
c) shaping the droplets in air or in a liquid which is not miscible with water, characterized in that
d) the obtained hydrogel particles are predried partially by removing between 45%w and 85%w of water, calculated on the amount of water initially present in the hydrogel,
e) the partially pre-dried hydrogel particles are subjected to a hydrothermal treatment at a temperature between 80°C and 350°C,
f) the cation content of the hydrogel particles thus treated is decreased in an aqueous medium to less than 10%w, calculated on dry material, and
g) these hydrogel particles are dried and optionally the silica particles thus obtained are calcined.

The silica hydrosol can be prepared conveniently by mixing an aqueous solution of an alkalimetal silicate with an aqueous solution of an acid. Suitable alkalimetal silicates comprise the so-called waterglasses, based on $Na_2O/SiO_2$ having a sodium:silicon molar ratio between 1 and 0.2. Suitable acids comprise hydrochloric acid, nitric acid and especially sulphuric acid. Good results have been obtained using a molar ratio in the range 0.5-1.2 acid/waterglass, especially using a molar ratio in the range 0.6-0.8 acid/waterglass. The reactants can be used in various molar concentrations. Preferably, waterglasses are used in molar concentrations between 0.5 and 1.3 and the acid can be chosen accordingly.

The process of mixing may be performed suitably by leading the starting solutions separately via capillaries into tubular mixing chambers. Thorough mixing which appears to be important to produce uniform particles is established by carefully controlling the flow conditions in and outside the appropriate nozzles. Very good results can be obtained using nozzles exhibiting specific dimensions as disclosed and claimed in British Patent Application No. 8104631.

After the silica hydrosol has been formed, it is converted into droplet form by carefully controlling the break-up of the emerging stream which allows the formation of particles of uniform predetermined size and uniform shape. This may be achieved by gelling the hydrosol in a liquid which is immiscible, or substantially immiscible with water such as an oil, e.g. a paraffinic oil. This may be performed suitably by introducing the hydrosol into the upper end of a vertically disposed tube filled with oil. Best results are obtained when a relatively short sol-gel transformation time is applied, e.g. less than 15 seconds. It is also possible to spray the droplets in air.

The gelled particles thus obtained may be caught in an aqueous phase such as water or, preferably, an aqueous solution of a salt such as sodium sulphate, particularly a salt solution having substantially the same salt concentration as that present in the hydrogel particles. The hydrogel particles are then separated from the aqueous phase, e.g. by filtration or centrifugation. It is also possible to separate them directly from the oil phase but that is more cumbersome in view of further steps in the preparation of the final particles. If desired the hydrogels obtained may be left for some time during which shrinkage of their volume may gradually occur. The hydrogel particles thus obtained contain large amounts of water and also contain, apart from silica, watersoluble sodium salts as well as chemically bonded sodium ions.

It will be appreciated that the process according to the present invention allows for the preparation of silica particles of various sizes and shapes such as spheres, granules and extrudates. The process is especially suitable for the preparation of silica spheres which combine a number of advantageous properties such as high intrinsic strength, virtually no formation of fines when used in fixed or moving bed catalytic reactions as well as optimal transport characteristics. As discussed hereinbefore, the present process allows not only particles, especially spheres of different sizes but also having at the same time similar pore volumes and yet different but uniform pore diameters and vice versa. For instance, porous structures suitable for hydrodemetallization normally have pore diameters which are several times higher than those used in the hydration of ethylene whereas the diameter of the particles may be the same and their pore volume may differ more than 100%. The combination of partially pre-drying the hydrogel particles obtained and a hydrothermal treatment adjusted for the type of product required produces final particles having optimal performance parameters.

It has been found that pure strong silica particles can be obtained when the amount of water present in the hydrogel is reduced very carefully, not only with respect to the temperature applied but also with respect to the amount of water which should remain in the then partially dried particles prior to the hydrothermal treatment. This is not only necessary to prevent the formation of cracks in the particles which might occur at forced or prolonged drying but also to determine the texture of the particles which is dependant on moisture content and temperature.

The amount of water to be removed from the hydrogel particles obtained should be between rather narrow ranges, e.g. between 45%w and 85%w, calculated on the amount of water initially present in the hydrogel. It has been found that very good results can be obtained when the amount of water remaining in the pre-dried particles is between 12%w and 70%w, especially between 30%w and 50%w, calculated on the total weight of the pre-dried particles.

The partial pre-drying of the hydrogel particles can be carried out by methods known in the art, provided that forced and prolonged drying, i.e. at excessive temperatures or to too high a degree of water removal are avoided. For instance, water may be removed from the hydrogel particles by contacting them with a gas stream, e.g. a stream of air, either or not at elevated temperature. Water may also be removed by heating the hydrogel particles carefully at atmospheric pressure or at reduced or elevated pressure. Water may also be removed from the hydrogel particles by contacting them with an inert liquid at a temperature above 100°C, although the inert liquid will replace at least part of the water which causes problems during further process steps, or by contacting them with steam or a steam-containing gas stream.

Partial drying can be carried out conveniently by blowing air over the hydrogel particles either at constant temperatures or at slowly increasing temperatures provided that not even a small amount of the hydrogel particles are dried to completion. Temperatures ranging from ambient to 250°C can be used, preference being given to temperatures in the range of from 70°C to 180°C. It appears to be especially the amount of water remaining in the partially dried hydrogel which determines the pore volume. The flexibility of the process according to the present invention is particularly vested in the unique property that once the pore volume has been set, the pore diameter can still be chosen. This allows, for instance, the preparation of silica particles having large pore volumes, e.g. > 1.2 ml/g as well as large pore diameters, e.g. even as high as 70 nm or even higher. The advantage of particles having large pore volumes is that higher loading of catalytically active materials can be applied. This is of particular interest in hydrodemetallization processes as well as for the hydration of ethylene over a carrier impregnated with phosphoric acid.

As discussed herein before, the pre-dried silica particles are next subjected to a hydrothermal treatment, i. e. a treatment at elevated temperature with liquid water and/or water vapour. This treatment results in a controllable growth of the pore diameter whilst the pore volume is substantially retained. It is necessary to perform the hydrothermal treatment in the presence of one or more compounds selected from the group of compounds of the elements Li, Na, K, Cs, Rb, Ca, Sr, Ba as well as $NR^1R^2R^3R^4$ compounds wherein $R^1$, $R^2$, and $R^3$ and $R^4$ which may be the same or different each represent a hydrogen atom or a hydrocarbyl group, e.g. an alkyl group having up to 12 carbon atoms, and $NH_3$.

Since the partially-dried hydrogels still contain alkali ions, there is no special need to add one or more of the compounds mentioned hereinbefore. In the event that the alkalimetal salts had been removed from the hydrogel, fresh amounts of such compound or of any compound referred to hereinabove would have to be added prior to the hydrothermal treatment.

When the hydrothermal treatment is effected by treating the partially dried silica particles with liquid water at elevated temperature, in general a treating temperature between 50°C and 374°C is chosen. Preferred treating temperatures are between 80°C and 350°C and in particular between 100°C and 300°C. When using a treating temperature above 100°C, the treatment has to be carried out in a closed vessel under autogenous pressure. The treating times generally range between 15 minutes and 24 hours. The volume of liquid water to be applied is preferably chosen such that during the treatment the partially-dried silica particles are completely surrounded by water. When using treating temperatures below 100°C it is sufficient to employ a quantity of liquid water substantially equal in volume to that of the silica particles to be treated. This also holds when the treatment is carried out at a temperature above 100°C in a closed vessel under autogenous pressure, provided that the volume of the closed vessel is sufficiently larger than the volume of the silica particles and the volume of water to be applied. When the treatment is carried out at a temperature above 100°C in a closed vessel whose volume is considerably larger than twice the volume of the silica particles to be treated, a larger volume of liquid water has to be used.

When the hydrothermal treatment is carried out by treating the partially-dried silica particles with water vapour at an elevated temperature, the treating temperature is generally chosen between 100°C and 500°C, preference being given to temperatures in the range of from 100°C to 300°C. The water partial pressure applied is preferably chosen between 1 and 40 bar. The heating times generally range between 15 minutes and 24 hours. The treatment may be effected either by keeping the silica particles in a closed vessel in contact with a certain quantity of saturated water vapour or by continuously passing saturated water vapour over the silica particles. In the latter case it is preferred to use a linear gas velocity between 1 and 100 cm/h.

The amount of one or more compounds selected from the group of compounds of the elements Li, Na, Cs, Rb, Ca, Sr, Ba as well as $NR^1R^2R^3R^4$-compounds and $NH_3$ present in the partially-dried silica particles to be subjected to hydrothermal treatment may vary between wide ranges, e.g. an amount of from 01. gram of the compound concerned up to 25 g of the compound per 100 gram of the silica in the particles to be hydrothermally treated. Preference is given to the use of compounds in the amount of from 0.5 to 15 g per 100 g of silica in the particles to be treated. Good results have been obtained using silica particles still containing part or all of the alkali ions, e.g. sodium ions present therein because of the formation of the hydrosol as discussed hereinbefore.

The process according to the present invention thus combines the possibility of primarily setting the pore volume by a controlled partial drying of the hydrogel and of primarily setting the pore diameter by a hydrothermal treatment which make it very flexible with respect to the silica to be prepared.

The hydrothermal treatment will be followed by a treatment to decrease the cation content of the silica particles to less than 10%w, preferably less than 5%w, calculated on dry material. Normally, the cations present are sodium ions originating from the waterglass constituent in the formation of the hydrosol. It is also possible that the cations are present since they were added to facilitate the hydrothermal treatment.

The decrease in the amount of cation can be conveniently performed by washing the silica particles one or several times with water so that the concentration is reduced to the desired level. Depending on the intended use of the final silica particles the amount of cation may be reduced to, say, 7% or less, calculated on dry material when an alkaline carrier is desired, or to less than 1%w when neutral carriers are required. In the latter case it may be advantageous to subject the silica particles to a treatment with an inorganic or organic acid in order to remove cation bound to the silica particles themselves. Also aqueous solutions of ammonium salts, e.g. ammonium nitrate can be used to reach the desired low alkalimetal content of the silica particles. It should be noted that ammonium ions, which have replaced any cations bound to the silica particles will decompose during the subsequent drying and calcining treatment so that the final product does contain the desired amount of cations, if any.

Finally the hydrogel particles thus obtained are dried and calcined. Drying may be carried out by methods known in the art. Since the structure parameters have been firmly set by the process according to the present invention, the final drying conditions appear not to be crucial. Since water has to be removed again the same methods described hereinbefore can be used conveniently. For instance, the hydrogel particles can be dried smoothly by heating them at a temperature of about 100°C at reduced pressure or by heating them at a temperature above 100°C in a stream of air. But other methods are also applicable. Normally drying is carried out for several hours at temperatures up to 200°C.

Calcining may be carried out by methods known in the art. It will be appreciated that higher calcining temperatures have to be used when the dried hydrogel contains ammonium ions as described hereinbefore. The temperature at which the calcination occurs may vary between wide ranges. Normally temperatures up to 600°C can be used but higher temperatures are not excluded. It may even be advantageous for certain applications, such as hydration reactions to subject the dried hydrogel to a calcination at a temperature up to 1000°C, preferably between 800°C and 950°C. Normally calcining will be carried out during relatively short periods, e. g. periods of up to one hour, but longer periods may also be used.

The novel silica particles, and especially silica spheres, are characterized by a very high attrition resistance. When the silica spheres are subjected to a standard attrition test as described in Example 9, the loss on attrition is less than 0.1%w and normally even below 0.05%w, i.e.< 50 mg/kg. The near perfect shape of the silica spheres and their well-controlled structure allow for the unusually low loss on attrition which can be achieved even without the use of specific attrition-resistance improving compounds such as graphite or aluminium.

The novel silica particles, and especially silica spheres are further characterized in that they exhibit a very narrow pore size distribution at any set pore volume. This is of course advantageous since many chemical reactions can be performed much better when uniform carriers can be used. This very narrow pore size distribution holds both for relatively small and relatively large pore diameters. It has been found that silica spheres having a mean pore diameter of not more than 30 nm (as determined by mercury porosimetry, as described by H. L. Ritter and L. C Drake Ind. Eng. Chem., Anal. Edition *17* pp. 787 (1945), applying mercury pressures of 1 to 2000 bar) exhibit a pore size distribution wherein at least 70% of the pore volume is made up of pores having pore diameters which have a tolerance of not more than 10 nm on the mean pore diameter and at least 60% of the pore volume is made up of pores having pore diameters which have a tolerance of not more than 5 nm on the mean pore diameter and that silica spheres having a mean pore diameter of more than 30 nm (as determined by mercury porosimetry) exhibit a pore size distribution wherein at least 65% of the pore volume is made up of pores having pore diameters which have a tolerance of not more than 20 nm on the mean pore diameter and at least 55% of the pore volume is made up of pores having pore diameters having a tolerance of not more than 10 nm on the mean pore diameter. Preferably, silica spheres having a mean pore diameter of not more than 30 nm (as determined by mercury porosimetry) exhibit a pore size distribution wherein at least 75% of the pore volume is made up of pores having pore diameters which have a tolerance of not more than 10 nm on the mean pore diameter and at least 65% of the pore volume is made up of pores having pore diameters which have a tolerance of not more than 5 nm on the mean pore diameter and silica spheres having a mean pore diameter of more than 30 nm (as determined by mercury porosimetry) exhibit a pore size distribution wherein at least 75% of the pore volume is made up of pores having pore diameters which have a tolerance of not more than 20 nm on the mean pore diameter and at least 65% of the pore volume is made up of pores having a tolerance of not more than 10 nm on the mean pore diameter. The pore volume referred to hereinabove is the total pore volume as measurable by the method of Innes (Analytical Chemistry 28, No. 3 (March 1956)).

When plotting the pore diameter (nm) as a function of the cumulative pore volume (ml/g) a curve is found which is substantially vertical.

The novel silica particles, and especially silica spheres are further believed to exhibit quite specific tortuosity factors. Tortuosity factors are expressed as the average length of the pass of a flowing gas relative to the shortest possible pass. (A.I.Ch.E. Journal, November 1968, pp. 886-895).

It will be understood that a very regular pore structure will contribute significantly to a relatively small tortuosity factor whereas irregular pores tends to contribute to relatively large tortuosity factors.

As discussed hereinbefore, the silica particles according to the present invention may be applied e.g. as catalysts, catalyst carriers, absorbents, drying agents and ion exchangers. They are of particular importance as carriers for one or more metals or metal compounds with catalytic activity. Catalysts comprising the present silica particles as carrier may be applied in various processes in the chemical and petroleum industries. The preparation of the catalysts may be carried out by any technique for the preparation of supported catalysts known in the art, e.g. by impregnating the silica particles with an aqueous solution comprising salts of the catalytically active metals concerned, followed by drying and calcining of the composition. A suitable way of preparing the present catalysts is one in which the catalytically active metals are incorporated into the carrier in an early stage of the carrier preparation, e.g. when the latter is still in the hydrogel form. This may have an impact on the porosity of the ultimate carrier to be made and it also renders the additional drying and calcining steps required after separate impregnation optional.

Silica particles, and particularly silica spheres prepared according to the process according to the present invention are of particular importance as carriers for catalysts which are normally used in the hydrodemetallization of heavy hydrocarbon oils, the epoxidation of olefinically unsaturated compounds with organic hydroperoxides and in the hydrations of olefinically unsaturated compounds to produce the corresponding alkanols.

Hydrodemetallization of heavy hydrocarbon oils is a well-known process in the petroleum industry and is applied, amongst other things, to decrease the metal content of heavy hydrocarbon oils which are to be used as feed for catalytic treating processes such as hydrodesulphurization and catalytic cracking. As a result of the demetallization, the life of the catalyst in the subsequent treating or conversion process is prolonged. Hydrodemetallization is carried out by contacting the heavy hydrocarbon oil at elevated temperature and pressure in the presence of hydrogen with a catalyst. Preferred catalysts for this purpose are catalysts comprising one or more metals selected from the group consisting of nickel, cobalt, molybdenum, tungsten and vanadium on a silica carrier. Especially preferred are catalysts comprising at least one metal selected from the group consisting of nickel and cobalt and at least one metal selected from the group consisting of molybdenum, tungsten and vanadium, such as the metal combinations nickel/vanadium, nickel/ molybdenum and cobalt/molybdenum on a silica carrier. Silica spheres prepared according to the process according to the present invention are preferred carriers for these catalysts because they exhibit higher metal loadings because of their specific structure and texture.

Epoxidation of olefinically unsaturated compounds with an organic hydroperoxide is a well-known process in the chemical industry and is applied for instance for the manufacture of propylene oxide and epichlorohydrin from propylene and allyl chloride, respectively. The epoxidation of olefinically unsaturated compounds with an organic hydroperoxide is carried out by contacting the reactants preferably at elevated temperature and pressure with a catalyst. As organic hydroperoxide, preference is given to ethyl benzene hydroperoxide since methyl phenylcarbinol is obtained as a by-product in the epoxidation which compound can be easily converted into valuable styrene. Preferred catalysts for the epoxidation are catalysts comprising at least one metal or metal compound selected from the group consisting of molybdenum, tungsten, titanium, zirconium and vanadium on a silica carrier. Especially preferred are catalysts comprising titanium on a silica carrier. The silica spheres prepared according to the process according to the present invention can be used advantageous as carriers for these catalysts.

The silica particles can also be suitably applied as carriers for catalysts used in the puerification of exhaust gases. They can also be used as carriers for certain polymerization and oligomerization catalysts for lower olefins, e.g. as carriers for the phosphoric acid catalyzed oligomerization of ethylene. The catalysts normally suggested for this type of reactions can be suitably used on the silica particles.

The hydration of olefinically unsaturated compounds, using a supported acidic catalyst, is a well-known process in the chemical industry. Suitable olefins are those containing from 2 to 10 carbon atoms, especially those having from 2 to 5 carbon atoms. Most preferred are ethylene and propylene.

The reactants are generally applied in the gaseous state. The feed ratio and the reaction conditions may vary widely, depending inter alia on the starting material used. Thus, ethanol may suitably be prepared using a molar ratio water/ ethylene in the range 0.2:1 to 1.0:1, preferably 0.3:1 to 0.6:1, a temperature in the range 200°C to 300°C, preferably 220°C to 270°C and a pressure of from 50 to 90 bar. Isopropylalcohol may be prepared, for example, using a molar ratio water/ propylene ranging from 0.1:1 to 0.5:1 a temperature of from 140°C to 250°C, and a pressure in the range from 15 to 50 bar. The hydration catalyst based on a silica carrier may be prepared by means of any conventional technique. According to a preferred method, silica particles, e.g. silica spheres, prepared as described hereinbefore are impregnated with phosphoric acid. Suitably an aqueous phosphoric acid is used having a concentration of, for example, from 20 to 85%, preferably from 50 to 75%. Good results are obtained by immersion of the silica particles in the aqueous phosphoric acid, e.g. from 0.5 to 5 hours, followed by draining off the excess and drying the impregnated support in the usual way, for example by heating at about 150°C.

The invention will now further be elucidated with the aid of the following Examples.

Example 1

An aqueous sodium waterglass solution comprising 12%w $SiO_2$ and having a $Na_2O/SiO_2$ molar ratio of 0.3 was mixed continuously in a mixing chamber with an aqueous 1.2 N sulphuric acid solution in a volume ratio acid solution/waterglass of 0.65. After a residence of a few seconds in the mixing chamber the hydrosol obtained was converted into droplet form and the hydrosol droplets allowed to fall through a vertically disposed cylindrical tube with a length of 1.8 m filled with a paraffinic hydrocarbon oil at 25°C. During the fall through the tube gelation occurred. The globular hydrogel particles were caught at the bottom of the tube in water of 25°C. After the globular hydrogel particles had been separated by filtration they were washed with water. The water content of the hydrogel particles prior to further treatment amounted to 90%w, as determined using a standard-test (heating a sample in three hours from ambient temperature to 600°C and thereafter keeping the same thus heated at 600°C for one hour).

A part of the hydrogel particles thus obtained (total volume 120 ml) was then subjected to a careful partial drying procedure by drying with air of relatively high velocity (1.5-2.0 m/s) at a temperature between 80°C and 90°C until the moisture content of the hydrosol particles had reached a value between 35%w and 50%w which normally amounted to driving times up to 20 minutes. The quality of the spheres thus treated was good (virtually no fines).

Thereafter the hydrosol particles thus treated were subjected to an hydrothermal treatment in an autoclave. An amount of water was added so that the volume ratio of water:bulk hydrogel particles (partially dried) was 0.7. This mixture was heated in 4 hours to 180°C and then kept at this temperature for 2 hours whereafter the mixture was cooled down gradually. The hydrogel particles thus treated were treated with an aqueous solution of ammonium nitrate at ambient temperature until the sodium content of the particles had been decreased to less than 0.2%w, calculated on dry material. After drying for 2 hours at 150°C the silica spheres were subjected to a final calcination at 500°C during 3 hours. The silica spheres thus obtained had still good properties. The pore volume as determined by mercury porosimetry amounted to 1.32 ml/g and the pore diameter as determined by mercury porosimetry amounted to 90 nm with an unusually narrow pore diameter distribution.

When the experiment was repaired but allowing a storage time of 16 hours between the partial drying and the hydrothermal treatment, no difficulties were encountered.

Example 2

The experiment as described in Example 1 was repeated using a sulphuric acid/waterglass volume ratio of 0.70. Having subjected the hydrogel particles to the same treatments as described in Example 1, good quality silica spheres were obtained having a pore volume of 1.24 ml/g and a pore diameter of 88 nm.

Example 3

The experiment as described in Example 2 was repeated using a sulphuric acid/waterglass volume ratio of 0.75. Having subjected the hydrogel particles to the same treatments as described in Example 2, good quality silica spheres were obtained having a pore volume of 1.48 ml/g and a pore diameter of 75 nm.

Example 4

Silica spheres having a size range between 2.3-2.8 mm were produced using the procedure described in Example 1 wherein partial drying with air was carried out until the moisture content of the hydrogel particle amounted to 0.72 kg/kg hydrogel (i.e. a water content of 41.8%w). Thereafter the hydrogel particles were subjected to a standard hydrothermal treatment by gradually heating them in the normal amount of water in an autoclave to 160°C and keeping the mixture at that temperature for 1 hour whereafter the mixture was cooled down slowly. Finally the hydrogel particles thus obtained were treated with an ammonium nitrate solution, washed and dried at 120°C. Silica spheres were obtained having a pore volume (Hg-porosimetry) of 1.12 ml/g and a pore diameter (Hg-porosimetry) of 41 nm. The amount of fines formed was about 0.05%w. The mean side crushing strength of the spheres obtained as determined by a standard test method amounted to 49.8 N. Cracked spheres, having a SCS < 10 N could not be detected. The water resistance of the silica spheres thus produced was 100% expressed as WR 10, i.e. the percentage of silica spheres which have not been damaged by the contact with a volume of water 5 times the volume of 10 silica spheres examined during 5 minutes.

Example 5

The experiment described in Example 4 was repeated to obtain silica spheres having a size range between 2.8-3.3 mm by using a mixing chamber having a larger aperture. The partial drying of the hydrogel particles was carried out in such a way that the moisture content of the particles amounted to 0.84 kg-kg hydrogel (i. e. a water content of 45.6%w). Thereafter the hydrogel particles were subjected to the hydrothermal treatment described in Example 4, treated with an ammonium nitrate solution, washed and finally calcined at 300°C. Silica spheres were obtained having a pore volume of 1.28 ml/g and a pore diameter of 45 nm. The amount of fines formed (5<2.38 mm) was 0.8%w. The mean side crushing strength was 42.7 N, no cracked spheres were detected and the water resistance, expressed as WR 10, was again 100%.

Example 6

Silica spheres having a size range between 1.4-1.7 mm were produced using a mixing chamber having a rather smaller aperture. The partial drying was carried out at 80-90°C until the water content in the partially dried hydrogel particle amounted to 0.23 kg/kg hydrogel. Thereafter, the hydrogel particles were subjected to a standard hydrothermal treatment by heating them immersed in water in an autoclave to 145°C and keeping the mixture at that temperature during 1 hour prior to cooling the mixture. After treatment with ammonium-nitrate, washing and drying, silica spheres were obtained having a pore volume (Hg-porosimetry) of 0.49 ml/g and a pore diameter of 22 nm.

Example 7

The experiment described in Example 6 was repeated with the proviso that the amount of water remaining in the partially dried hydrogel amounted to 0.63 kg/kg hydrogel. After the hydrothermal treatment and further process steps as described in Example 6, and calcination at 500°C during 1 hour, silica spheres were obtained having a pore volume of 0.98 ml/g and a mean pore diameter of 30 nm. The silica spheres thus prepared exhibit an extremely narrow pore size distribution curve. From the pV/pd graph it was calculated that more than 70% of the pore volume was made up of pores exhibiting pore diameters having a spread of not more than 5 nm on the mean pore diameter.

Example 8

Some experiments were carried out to determine the properties of the silica particles, and especially silica spheres prepared according to the process according to the present invention as carriers for catalysts.

A) Carriers for Hydrodemetallization Catalysts

Silica particles prepared as described in Example 5 were impregnated with salts of nickel and vanadium according to well-known procedures described, inter alia, in British Patent Specification 1,438,645. A ready catalyst was obtained in the form of spherical particles having a size range between 2.8-3.3 mm, a pore volume of 0.89 ml/g and a pore diameter of 45 nm. The side crushing strength of the catalyst was 58 N. The bulk crushing strength (BCS) was 1.55 MPa. When compared with a commercially available carrier (size 2-3.3 mm, pore volume 1.00 ml/g, pore diameter 5 nm, SCS 22.3 N and BSC > 1.55 MPa) the pore diameter distribution of the silica particles prepared according to the process according to the invention and loaded with Ni and V was extremely narrow: more than 70% of the pore volume was made up of pores exhibiting pore diameters having a spread of not more than 10 nm on the mean pore diameter, whereas the commercially available carrier also loaded with about the same amount of Ni and V, showed a spread no less than 275 nm. It will be appreciated that silica spheres having even larger pore volume than disclosed in this example, e.g. pore volumes of 1.2 ml/g or even higher can be regarded as having a potentially higher uptake capacity in hydrodemetallization processes.

B) Carriers for Phosphoric Acid Catalysed Hydration of Olefins

The silica particles prepared according to the process according to the present invention can be used suitably as carriers to produce highly active catalysts for the hydration of olefins to alkanols. Especially carriers with relatively large pore volumes, e.g. of at least 1.00 ml/g are desirable for a good catalytic performance. It will be appreciated that also the unique feature of a very regular pore size distribution contributes to the catalytic performance.

The catalysts may be prepared by means of any of the conventional techniques. According to a preferred method, silica particles, especially silica spheres prepared according to the process according to the present invention are impregnated with phosphoric acid. Suitably an aqueous phosphoric acid is used having a concentration of, for example, from 20 to 85%, preferably from 50 to 75%. Good results are obtained by immersion of the silica spheres in the aqueous phosphoric acid, e. g. for 0.5 to 5 hours, followed by draining off the excess acid and drying the impregnated support in the usual way, for example by heating at about 150°C.

It has been found that a calcination treatment at very high temperatures, e.g. between 850°C and 950°C contributes substantially to a carrier having a high strength stability which is very important under practical conditions. For instance, silica spheres prepared according to the process according to the present invention having a pore volume of 1.28 ml/g, a pore diameter of 18 nm and a SCS of 90 N were impregnated with aqueous phosphoric acid and processed as described hereinbefore to contain 57%w of phosphoric acid on catalyst. The thus loaded silica spheres were then subjected to true ethylene hydration conditions (temperature 260-280°C, water/ethylene mol ratio 0.4-0.8, pressure 80 bar) during one week. Thereafter the catalyst properties were determined again. The pore volume amounted to 1.00 ml/g, the pore diameter had increased to 200 nm and the SCS decreased to 16.3 N. Again it was found that they compare favourable with commercially available carriers which had been impregnated and subjected to a simulated hydration process.

Example 9

The attrition resistance of the silica particles prepared according to the process according to the present invention was determined using a standard attrition test according to the Peter Spence method. A portion of about 25 g of silica particles not showing any visible defections was weighed out to the nearest 50 mg and placed in an attrition tube which comprises an iron tube of internal length 305 mm and internal diameter of 33 mm which can rotate at 25 rpm about an axis normal to the length and at a point approximately 75 mm from the center of the tube. The iron tube containing the particles is then rotated at 25 rpm during 1 hour. Thereafter the material was sieved off over a sieve having a mesh width of 3.35 mm and the loss on attrition calculated by means of the equation

$$L = \frac{(W - A)^{\cdot}}{W} \times 100$$

wherein L is the loss on attrition in %w, A is the weight of the particles after rotating in grams and W is the weight of the particles-before rotating in grams. The silica spheres prepared according to the process according to the present invention did not shown any detectable loss on attrition at all whereas commercially available silica particles showed a loss well over 0.5%w.

**Claims**

1. Process for the preparation of silica particles by

a) preparing a silica hydrosol by mixing an aqueous solution of an alkali metal silicate with an aqueous solution of an acid,

b) converting the hydrosol into droplet form,

c) shaping the droplets in air or in a liquid which is not miscible with water, characterized in that

d) the obtained hydrogel particles are predried partially by removing between 45%w and 85%w of water, calculated on the amount of water initially present in the hydrogel,

e) the partially pre-dried hydrogel particles are subjected to a hydrothermal treatment at a temperature between 80°C and 350°C,

f) the cation content of the hydrogel particles thus treated is decreased in an aqueous medium to less than 10%w, calculated on dry material, and

g) these hydrogel particles are dried and optionally the silica particles thus obtained are calcined.

2. Process according to claim 1 characterized in that the hydrothermal treatment is carried out using a quantity of liquid water at least substantially equal in volume to that of the silica particles to be treated.

3. Silica particles, prepared by the process of claim 1 characterised by having a pore size distribution wherein at least 70% of the pore volume is made up of pores having pore diameters which have a tolerance of not more than 10 nm on the mean pore diameter and at least 60% of the pore volume is made up of pores having pore diameters which have a tolerance of not more than 5 nm on the mean pore diameter when the mean pore diameter is not more than 30 nm (as determined by mercury porosimetry) and having a pore size distribution wherem at least 65% of the pore volume is made up of pores having pore diameters which have a tolerance of not more than 20 nm on the mean pore diameter and at least 55% of the pore volume is made up of pores having pore diameters which have a tolerance of not more than 10 nm on the mean pore diameter when the mean pore diameter is more than 30 nm (as determined by mercury porosimetry), and by having a loss on attrition of less than 0.1% as determined by the Peter spence method placing 25g of silica in an iron attrition tube of 305 mm internal length an 33 mm internal diameter and rotating the tube for 1 hour at 25 rpm about an axis normal to the length and at a point approximately 75 mm from the center of the tube, and calculating after sieving over a sieve having a mesh width of 3.35 mm, the loss on attrition by means of the equation $L = \frac{(W-A)}{W} \times 100$ wherein L is the loss on attrition in % w, A is the weight of the particles after rotating in grams and W is the weight of the particles before rotating in grams.

4. Silica particles according to claim 3, characterised by having a pore size distribution wherein at least 75% of the pore volume is made up of pores having pore diameters which have a tolerance of not more than 10 nm on the mean pore diameter and at least 65% of the pore volume is made up of pores having pore diameters which have a tolerance of not more than 5 nm on the mean pore diameter when the mean pore diameter is not more than 30 nm and at least 75% of the pore volume is made up of pores having pore diameters which have a tolerance of not more than 20 nm on the mean pore diameter and at least 65% of the pore volume is made up of pores having pore diameters which have a tolerance of not more than 10 nm on the mean pore diameter when the mean pore diameter is more than 30 nm.

5. Catalysts comprising one or more metals or metal compounds with catalytic activity on silica particles as claimed in claim 3 as carrier.

6. Process for the hydrometallisation of a heavy hydrocarbon oil wherein the heavy oil is contacted at elevated temperature and pressure and in the presence of hydrogen with a catalyst according to claim 5, which catalyst comprises at least one metal selected from the group consisting of nickel and cobalt and at least one metal selected from the group consisting of molybdenum, tungsten and vanadium as catalytically active metals.

7. Process for the epoxidation of olefinically unsaturated compounds with an organic hydroperoxide wherein the reactants are contacted with a catalyst according to claim 5, which catalyst comprises at least one metal or metal compound selected from the group consisting of molybdenum, tungsten, titanium, zirconium and vanadium as catalytically active compounds.

8. Process for the purification of exhaust gases wherein the exhaust gases are contacted with a catalyst according to claim 5.

9. Process for the hydration of olefinically unsaturated compounds wherein the reactants are contacted with a catalyst according to claim 5, which catalyst comprises phosphoric acid.

**Patentansprüche**

1. Verfahren zur Herstellung von Siliciumdioxidteilchen durch

a) Herstellen eines Siliciumdioxid-Hydrosols durch Vermischen einer wässrigen Lösung eines Alkalimetallsilikats mit einer wässrigen Lösung einer Säure,

b) Umwandeln des Hydrosols in Tröpfchenform,

c) Formgebung der Tröpfchen in Luft oder in einer mit Wasser nicht mischbaren Flüssigkeit,

dadurch gekennzeichnet, daß

d) die so erhaltenen Hydrogelteilchen durch Entfernen von 45 bis 85 Gewichtsprozent Wasser, bezogen auf die ursprünglich im Hydrogel enthaltene Wassermenge, teilweise vorgetrocknet werden,

e) die teilweise vorgetrockneten Hydrogelteilchen bei einer Temperatur zwischen 80°C und 350°C einer Hydrothermalbehandlung unterworfen werden,

f) der Gehalt an Kationen der so behandelten Hydrogelteilchen in einem wässrigen Medium auf weniger als 10 Gewichtsprozent, bezogen auf trockenes Material, verringert wird, und

g) diese Hydrogelteilchen getrocknet und die dabei erhaltenen Siliciumdioxidteilchen gegebenenfalls calciniert werden.

2. Verfahren nach Anspruch 1, dadurch gekennzeichnet, daß die Hydrothermalbehandlung unter Verwendung einer solchen Menge an flüssigem Wasser durchgeführt wird, welche mindestens im wesentlichen der Volumenmenge der zu behandelnden Siliciumdioxidteilchen entspricht.

3. Siliciumdioxidteilchen, hergestellt nach dem Verfahren gemäß Anspruch 1, dadurch gekennzeichnet, daß sie eine Porengrößenverteilung aufweisen, in welcher mindestens 70% des Porenvolumens aus Poren mit einem Porendurchmesser besteht, welche eine Toleranz von nicht mehr als 10 nm, bezogen auf den mittleren Porendurchmesser, aufweisen und bei denen mindestens 60% des Porenvolumens aus Poren mit Porendurchmessern besteht, die eine Toleranz von nicht mehr als 5 nm bezogen auf den mittleren Porendurchmesser, aufweisen, wenn der mittlere Porendurchmesser nicht größer als 30 nm ist (bestimmt nach der Quecksilber-Porositätsmessung) und welche eine Porengrößenverteilung derart aufweisen, daß mindestens 65% des Porenvolumens aus Poren mit einem Porendurchmesser bestehen, welche eine Toleranz von nicht mehr als 20 nm, bezogen auf den mittleren Porendurchmesser, aufweisen, und mindestens 55% des Porenvolumens aus Poren mit einem Porendurchmesser bestehen, welche eine Toleranz von nicht mehr als 10 nm, bezogen auf den mittleren Porendurchmesser, aufweisen, falls der mittlere Porendurchmesser größer als 30 nm ist (bestimmt mittels der Quecksilber-Porositätsmessung), und daß sie einen Abriebverlust von weniger als 0,1 Gewichtsprozent aufweisen, bestimmt gemäß der Methode von Peter Spence, wobei 25 g Siliciumdioxid in ein Eisenabriebrohr von 305 mm Innenlänge und 33 mm Innendurchmesser eingebracht werden, das Rohr 1 Stunde lang mit 25 Umdrehungen je Minute um eine Achse rotieren gelassen wird, die senkrecht zur Längserstreckung und an einem Punkt von etwa 75 mm von der Mitte des Rohres verläuft, und nach einem Sieben über ein Sieb mit einer Maschenweite von 3,35 mm der Abrieb nach der Formel $L = \frac{(W - A)}{W} \cdot 100$ berechnet wird, worin L der Abrieb in Gew.-%, A das Gewicht der Teilchen in Gramm nach dem Rotieren und W das Gewicht der Teilchen in Gramm vor dem Rotieren ist.

4. Siliciumdioxidteilchen gemäß Anspruch 3, dadurch gekennzeichnet, daß sie eine Porengrößenverteilung aufweisen, in welcher mindestens 75% des Porenvolumens aus Poren mit Porendurchmessern bestehen, die eine Toleranz von nicht mehr als 10 nm, bezogen auf den mittleren Porendurchmesser, aufweisen, und mindestens 65% des Porenvolumens aus Poren mit Porendurchmessern bestehen, welche eine Toleranz von nicht mehr als 5 nm, bezogen auf den mittleren Porendurchmesser, haben, falls der mittlere Porendurchmesser nicht größer als 30 nm ist, und bei der mindestens 75% des Porenvolumens aus Poren mit Porendurchmessern bestehen, welche eine Toleranz von nicht mehr als 20 nm, bezogen auf den mittleren Porendurchmesser, haben, und mindestens 65% des Porenvolumens aus Poren mit Porendurchmessern bestehen, welche eine Toleranz von nicht mehr als 10 nm, bezogen auf den mittleren Porendurchmesser, haben, falls der mittlere Porendurchmesser größer als 30 nm ist.

5. Katalysatoren, umfassend ein oder mehrere Metalle oder Metallverbindungen mit katalytischer Aktivität auf Siliciumdioxidteilchen gemäß Anspruch 3 als Trägermaterial.

6. Verfahren zur hydrierenden Entmetallisierung eines schweren Kohlenwasserstofföls, in welchem das schwere Öl bei erhöhter Temperatur und Druck in Gegenwart von Wasserstoff mit einem Katalysator gemäß Anspruch 5 kontaktiert wird, welcher Katalysator mindestens ein Metall, ausgewählt aus der Gruppe, bestehend aus Nickel und Kobalt, und mindestens ein Metall, ausgewählt aus der Gruppe von Molybdän, Wolfram und Vanadium, als katalytisch aktive Metalle umfaßt.

7. Verfahren zur Epoxidierung von olefinisch ungesättigten Verbindungen mit einem organischen Hydroperoxid, in welchem die Reaktanten mit einem Katalysator gemäß Anspruch 5 kontaktiert werden, welcher Katalysator mindestens ein Metall oder eine Metallverbindung, ausgewählt aus der Gruppe bestehend aus Molybdän, Wolfram, Titan, Zirkon und Vanadium, als katalytisch aktive Verbindungen umfaßt.

8. Verfahren zur Reinigung von Abgasen, in welchem die Abgase mit einem Katalysator gemäß Anspruch 5 kontaktiert werden.

9. Verfahren zur Anlagerung von Wasser an olefinisch ungesättigte Verbindungen, in welchem die Reaktanten mit einem Katalysator gemäß Anspruch 5 kontaktiert werden, welcher Katalysator Phosphorsäure umfaßt.

## Revendications

1. Procédé de préparation de particules de silice qui consiste à :

    a) préparer un hydrosol de silice en mélangeant une solution aqueuse d'un silicate de métal alcalin avec une solution aqueuse d'un acide,
    b) transformer l'hydrosol en gouttelettes,
    c) former les gouttelettes dans l'air ou dans un liquide qui ne soit pas miscible à l'eau, caractérisé en ce que d) les particules d'hydrogel obtenues sont préséchées partiellement par élimination d'entre 45% en poids et 85% en poids d'eau, relativement à la quantité d'eau initialement présente dans l'hydrogel,
    e) les particules d'hydrogel partiellement préséchées sont soumises à un traitement hydrothermique à une température comprise entre 80°C et 350°C,
    f) la teneur en cations des particules d'hydrogel ainsi traitées est réduite dans un milieu aqueux jusqu'à moins de 10% en poids, relativement à la matière sèche, et
    g) ces particules d'hydrogel sont séchées et, éventuellement, les particules de silice ainsi obtenues sont calcinées.

2. Procédé selon la revendication 1, caractérisé en ce que l'on réalise le traitement hydrothermique en utilisant une quantité d'eau liquide au moins pratiquement égale, en volume, à celle des particules de silice à traiter.

3. Particules de silice préparées par le procédé selon la revendication 1, caractérisées en ce qu'elles ont une distribution des grosseurs de pores dans laquelle au moins 70% du volume des pores est constitué de pores ayant des diamètres de pores présentant un écart d'au plus 10 nm par rapport au diamètre moyen des pores, et au moins 60% du volume des pores est constitué de pores ayant des diamètres de pores présentant un écart d'au plus 5 nm par rapport au diamètre moyen des pores, lorsque le diamètre moyen des pores ne dépasse pas 30 nm (déterminé par porosimétrie au mercure), et en ce qu'elles ont une distribution des grosseurs de pores dans laquelle au moins 65% du volume des pores est constitué de pores ayant des diamètres de pores présentant un écart d'au plus 20 nm par rapport au diamètre moyen des pores, et au moins 55% du volume des pores est constitué de pores ayant des diamètres de pores présentant un écart d'au moins 10 nm par rapport au diamètre moyen des pores, lorsque le diamètre moyen des pores est supérieur à 30 nm (déterminé par porosimétrie au mercure) et en ce qu'elles possèdent une perte par abrasion inférieure à 0,1%, comme déterminé par le procédé de Peter Spencer en plaçant 25 g de silice dans un tube d'abrasion en fer d'une longueur interne de 305 mm, un diamètre interne de 33 mm et en faisant tourner le tube pendant 1 heure à 25 tpm autour d'un axe normal par rapport à la longueur et en un point situé approximativement à 75 mm du centre du tube et en calculant après tamisage sur un tamis ayant une largeur des mailles de 3,35 mm, la perte par abrasion à l'aide de l'équation $L = \frac{(W - A)}{W}$ x 100, où L est la perte par abrasion en % en poids, A est le poids des particules après rotation en grammes et W est le poids des particules avant rotation en grammes.

4. Parties de silice selon la revendication 3, caractérisées en ce qu'elles ont une distribution des grosseurs de pores dans laquelle au moins 75% du volume des pores est constitué de pores ayant des diamètres de pores présentant un écart d'au plus 10 nm par rapport au diamètre moyen des pores, et au moins 65% en volume des pores est constitué de pores ayant des diamètres de pores présentant un écart d'au plus 5 nm par rapport au diamètre moyen des pores, lorsque le diamètre moyen des pores ne dépasse pas 30 nm, au moins 75% du volume des pores est constitué de pores ayant des diamètres de pores présentant un écart d'au plus 20 nm par rapport au diamètre moyen des pores, et au moins 65% du volume des pores est constitué de pores ayant des diamètres de pores présentant un écart d'au plus 10 nm par rapport au diamètre moyen des pores, lorsque le diamètre moyen des pores est supérieur à 30 nm.

5. Catalyseurs comprenant un ou plusieurs métaux ou composés de métaux présentant une activité catalytique, sur des particules de silice comme support, préparées par le procédé selon la revendication 3.

6. Procédé d'hydrodémétallisation d'une huile hydrocarbure lourde, dans lequel on met en contact l'huile lourde, à une température et sous une pression élevées et en présence d'hydrogène, avec un catalyseur selon la revendication 5, ce catalyseur comprenant au moins un métal choisi dans le groupe constitué par le nickel et le cobalt et au moins un métal choisi dans le groupe constitué par le molybdène, le tungstène et le vanadium, comme métaux catalytique actifs.

7. Procédé d'époxydation de composés à insaturation oléfinique avec un hydroperoxyde organique, dans lequel on met en contact les réactifs avec un catalyseur selon la revendication 5, ce catalyseur comprenant au moins un métal ou un composé d'un métal choisi dans le groupe constitué par le molybdène, le tungstène, le titane, le zirconium et le vanadium, comme composés catalytiquement actifs.

8. Procédé de purification de gaz d'échappement, dans lequel les gaz d'échappement sont mis en contact avec un catalyseur selon la revendication 5.

9. Procédé d'hydratation de composés à insaturation oléfinique, dans lequel les réactifs sont mis en contact avec un catalyseur selon la revendication 5, ce catalyseur comprenant de l'acide phosphorique.